# EUROPEAN PATENT APPLICATION

(11) **EP 2 727 584 A1**
(43) Date of publication of application: **07.05.2014**
(21) Application number: 12190838.8
(22) Date of filing: 31.10.2012
(51) Int. Cl.: A61K 9/19, A61K 47/18, A61K 47/26, A61K 38/00, A61K 39/00, B65B 31/00, F26B 5/06, C07K 16/28

(54) **Vent gases for use in drying, storing and/or reconstituting biomolecules**

(71) Applicant: Takeda GmbH, 78467 Konstanz (DE)
(72) Inventor: Rast, Markus, 78315 Radolfzell (DE)
(74) Representative: Wild, Robert

(57) **Abstract**

The present invention relates to methods for treating a dry biomolecule allowing quick reconstitution of the dry biomolecule without excessive foam formation. Further, the invention relates to the use of a gas for reducing foam formation upon reconstitution of a dry biomolecule and a container comprising a dry biomolecule in a gas atmosphere capable of reducing foam formation upon reconstitution of a dry biomolecule.

## Description

### Field of the invention

The present invention relates to methods for treating a dry biomolecule allowing quick reconstitution of the dry biomolecule without excessive foam formation. Further, the invention relates to the use of a gas for reducing foam formation upon reconstitution of a dry biomolecule and a container comprising a dry biomolecule in a gas atmosphere capable of reducing foam formation upon reconstitution of a dry biomolecule.

### Background of the invention

Lyophilisation is used to increase the stability, temperature tolerance and shelf-life of biomolecules compared to an aqueous solution of the biomolecule. Most commonly, nitrogen is used in freeze-drying processes as an inert gas to control the pressure in the vials containing the dry biomolecules and to vent the vials prior to closing. For therapeutic applications the lyophilized biomolecules are usually reconstituted by dilution in a solvent. Highly concentrated solutions are often desired for therapeutic administration, such as subcutaneous administration. However, excessive foaming is often observed upon reconstitution of lyophilisates, especially for solutions having high concentrations of biomolecules like proteins or polysaccharides. This phenomenon leads to prolonged reconstitution times associated with difficulties for the health care personnel to judge whether reconstitution is complete.

### Summary of the invention

An object of the invention is to provide a method for quick reconstitution of a biomolecule avoiding excessive foam formation during the reconstitution process. It has been found that exposing the biomolecule during and/or after lyophilisation and until reconstitution to an atmosphere of carbon dioxide, nitrous oxide, hydrofluoroalkane and/or a mixture thereof avoids excessive foaming upon reconstitution and therefore accelerates the reconstitution step.

One aspect of the present invention concerns a method for treating a dry biomolecule. The method may comprise the following steps:
a) providing a dry biomolecule in a container,
b) exposing the dry biomolecule in the container to a gas selected from the group consisting of carbon dioxide, nitrous oxide, hydrofluoroalkane and/or mixtures thereof and
c) optionally, closing the container.

In one embodiment of the invention, the dry biomolecule is a freeze-dried biomolecule.

Typically, step (a) of the method for treating a dry biomolecule comprises freeze-drying the biomolecule.

In another embodiment, step (a) comprises providing the dry biomolecule in a container under a reduced gas pressure of 500 mbar or less, 300 mbar or less, or 200 mbar or less, or 100 mbar or less.

In a further embodiment, step (b) comprises exposing the dry biomolecule in the container to the gas so that the dry biomolecule is present in an atmosphere of the gas.

In an additional embodiment, step (c) comprises sealing the container so that the dry biomolecule is kept in the gas atmosphere.

Typically, the dry biomolecule is a protein.

In particular, the dry biomolecule may be an antibody.

Usually, the container is a vial, an ampoule, a carpule, a cartridge, a syringe or a double chamber system.

Typically, the hydrofluoroalkane is a tetrafluoroethane such as 1,1,1,2-tetrafluoroethane.

In another embodiment, the method for treating a dry biomolecule comprises a further step (d) of reconstitution, optionally with a solvent selected from the group consisting of water, buffer, physiological NaCl, Ringer solution, 5% glucose and/or PBS, in order to obtain a physiological acceptable solution.

Typically, the reconstituted biomolecule solution is isotonic.

In one embodiment, the reconstituted biomolecule solution is suitable for parenteral or oral administration to human patients.

Usually, the reconstituted biomolecule solution has a concentration of from between 30 mg/ml and 300 mg/ml, from between 60 mg/ml and 200 mg/ml or from between 80 mg/ml and 150 mg/ml.

Another aspect of the invention is a method for reconstituting a dry biomolecule, comprising the following steps:
a) providing a dry biomolecule in a gas atmosphere of a gas selected from the group consisting of carbon dioxide, nitrous oxide, hydrofluoroalkane and/or mixtures thereof,
b) adding a reconstitution solution to the dry biomolecule.

In a further embodiment, step (a) of the method for reconstituting a dry biomolecule comprises conducting the method for treating a dry biomolecule as described in any of the embodiments above.

Another aspect of the invention is the use of a gas selected from the group consisting of carbon dioxide, nitrous oxide, hydrofluoroalkane and/or mixtures thereof for reducing foam formation upon reconstitution of a dry biomolecule by adding a reconstitution solution to the dry biomolecule under an atmosphere of the gas.

A further aspect refers to a container, comprising a dry biomolecule in an atmosphere of a gas selected from the group consisting of carbon dioxide, nitrous oxide, hydrofluoroalkane and/or mixtures thereof.

### Detailed description of the invention

Where the term "comprise" or "comprising" is used in the present description and claims, it does not exclude other elements or steps. For the purposes of the present invention, the term "consisting of" is considered to be an optional embodiment of the term "comprising of". If hereinafter a group is defined to comprise at least a certain number of embodiments, this is also to be understood to disclose a group which optionally consists only of these embodiments.

Where an indefinite or a definite article is used when referring to a singular noun e.g. "a" or "an" or "the", this includes a plural form of that noun unless specifically stated. Conversely, when the plural form of a noun is used it refers also to the singular form. For example, when gases are mentioned, this is also to be understood as a single gas.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

One aspect concerns a method for treating a dry or solid biomolecule. The method may comprise the following steps:
a) providing a solid and/or dry biomolecule in a container,
b) exposing the solid and/or dry biomolecule in the container to a gas selected from the group consisting of carbon dioxide nitrous oxide, hydrofluoroalkane and/or mixtures thereof and
c) optionally, closing the container.

The term "biomolecule" includes molecules or polymers that may be produced by a living organism such as but not limited to proteins, peptides, oligopeptides, antibodies, polysaccharides, lipids, nucleic acids, primary metabolites, secondary metabolites, natural products, vitamins, hormones, neurotransmitters, nucleosides and nucleotides. Also chemically or post-translationally modified and fragment variants of these molecules are included. The term also includes vaccines or weakened or killed pathogens, such as bacteria or viruses, or a portion of a pathogen's structure which may have stimulating activity on the immune system against the pathogen but is incapable of causing severe infection. The biomolecule may be isolated from biological sources or made by methods involving chemical synthesis and/or *in vitro* reactions.

The term "hydrofluoroalkane" includes all alkanes which are gaseous under standard conditions for temperature and pressure (e.g. 25°C and atmospheric pressure) and wherein at least one hydrogen is substituted by fluorine. The term includes both gases consisting of one type of hydrofluoroalkane and gases consisting of a mixture of different types of hydrofluoroalkane. Exemplary hydrofluoralkanes are tetrafluorethane such as *inter alia* 1,1,1,2-tetrafluoroethane, also known as HFA 134a, HFC 134a, Solkane® 134a, and heptafluoropropane such as 1,1,1,2,3,3,3-heptafluoropropane, also known as HFA 227, HFC 227, or Solkane® 227.

Typically, a container may be *inter alia* a vial, an ampoule, a carpule, a cartridge, a syringe or a double chamber system.

Usually the dry or solid biomolecule in the container is exposed to a gas atmosphere by placing the container in a closed chamber that may be vented with one or more gases under an adjusted pressure. The chamber may be a freeze-dryer.

The dry or solid biomolecule may be exposed to a gas atmosphere of at least 50% carbon dioxide, such as at least 60% carbon dioxide, at least 70% carbon dioxide, at least 80% carbon dioxide, at least 95% carbon dioxide, at least 98% carbon dioxide or 100% carbon dioxide. In this embodiment, the gas atmosphere may also be designated as a carbon dioxide atmosphere.

Alternatively, the dry biomolecule may be exposed to a gas atmosphere of at least 50% nitrous oxide, such as at least 60% nitrous oxide, at least 70% nitrous oxide, at least 80% nitrous oxide, at least 95% nitrous oxide, at least 98% nitrous oxide or 100% nitrous oxide. In this embodiment, the gas atmosphere may also be denoted as a nitrous oxide atmosphere.

Alternatively, the dry biomolecule may be exposed to a gas atmosphere of at least 50% hydrofluoroalkane, such as at least 60% hydrofluoroalkane, at least 70% hydrofluoroalkane, at least 80% hydrofluoroalkane, at least 95% hydrofluoroalkane, at least 98% hydrofluoroalkane or 100% hydrofluoroalkane. In this embodiment, the gas atmosphere may also be denoted as a hydrofluoroalkane atmosphere.

Alternatively, the dry biomolecule may be exposed to a gas atmosphere of at least 50% of a mixture of carbon dioxide, nitrous oxide and hydrofluoroalkane, at least 60% of the mixture of carbon dioxide, nitrous oxide and hydrofluoroalkane, at least 70% of the mixture of carbon dioxide, nitrous oxide and hydrofluoroalkane, at least 80% of the mixture of carbon dioxide, nitrous oxide and hydrofluoroalkane or at least 95% of the mixture of carbon dioxide, nitrous oxide and hydrofluoroalkane, or 100% of the mixture of carbon dioxide, nitrous oxide and hydrofluoroalkane. The mixture may comprise any ratio of carbon dioxide, nitrous oxide and hydrofluoroalkane, such as 1: 1: 1, 2:1:1, 1:2:1, 1:1:2, 3:1:1, 1:3:1, 1:1:3, 1:2:3,3:2:1, 2:1:3, 2:3:1, 1:3:2 or 3:1:2.

Alternatively, the dry biomolecule may be exposed to a gas atmosphere of at least 50% of a mixture of carbon dioxide and nitrous oxide, at least 60% of the mixture of carbon dioxide and nitrous oxide, at least 70% of the mixture of carbon dioxide and nitrous oxide, at least 80% of the mixture of carbon dioxide and nitrous oxide or at least 95% of the mixture of carbon dioxide and nitrous oxide, or 100% of the mixture of carbon dioxide and nitrous oxide. The mixture may comprise any ratio of nitrous oxide and carbon dioxide, such as 5:1; 4:1; 3:1; 2:1; 1:1; 1:2; 1:3; 1:4 or 1:5.

Alternatively, the dry biomolecule may be exposed to a gas atmosphere of at least 50% of a mixture of carbon dioxide and hydrofluoroalkane, at least 60% of the mixture of carbon dioxide and hydrofluoroalkane, at least 70% of the mixture of carbon dioxide and hydrofluoroalkane, at least 80% of the mixture of carbon dioxide and hydrofluoroalkane or at least 95% of the mixture of carbon dioxide and hydrofluoroalkane, or 100% of the mixture of carbon dioxide and hydrofluoroalkane. The mixture may comprise any ratio of carbon dioxide and hydrofluoroalkane, such as 5:1; 4:1; 3:1; 2:1; 1:1; 1:2; 1:3; 1:4 or 1:5.

Alternatively, the dry biomolecule may be exposed to a gas atmosphere of at least 50% of a mixture of nitrous oxide and hydrofluoroalkane, at least 60% of the mixture of nitrous oxide and hydrofluoroalkane, at least 70% of the mixture of nitrous oxide and hydrofluoroalkane, at least 80% of the mixture of nitrous oxide and hydrofluoroalkane or at least 95% of the mixture of nitrous oxide and hydrofluoroalkane, or 100% of the mixture of nitrous oxide and hydrofluoroalkane. The mixture may comprise any ratio of nitrous oxide and hydrofluoroalkane, such as 5:1; 4:1; 3:1; 2:1; 1:1; 1:2; 1:3; 1:4 or 1:5.

During the venting the container may be partly closed with a lyo-stopper, such as a two-leg lyo stopper, three-leg lyo-stopper or igloo type lyo-stopper.

The term "closing" used herein interchangeably with the term "stoppering" refers to any procedure of closing the container for storage, for example with a cap, lid or foil. The term "dry biomolecule" or "solid biomolecule" or "biomolecule in dried form" or "biomolecule in solid form" or "dried biomolecule" or "biomolecule cake" or "cake" or "lyophilisate" or "lyophilisation cake" refers to any biomolecule that is not dissolved in a solvent or liquid. It may be present in powder or crystalline form. The dry protein in the above forms, e.g. as a lyphilisation cake, may contain stabilizers, bulking agents, osmolytes, buffering agents, detergents etc. In addition or alternatively, it may be produced by a drying method such as freeze-drying, spray-drying or evaporation. The term "dry biomolecule" may refer to a composition comprising a biomolecule having 10% water content or less, or 3% water content or less or 1% water content or less. In a specific preferred embodiment, the solid biomolecule is a freeze dried biomolecule.

In one embodiment step (a) of the method for treating a dry biomolecule comprises freeze-drying the biomolecule.

"Freeze-drying" also referred to as "lyophilization" or "cryodessiccation" denotes a drying process by freezing a biomolecule solution below 0°C and then reducing the surrounding pressure to allow the frozen water in the material to sublimate. Freeze-drying procedures are known in the art (see for example Kett V.P: et al. Cryo Letters 2004, Tang X & Pikal J., Pharmaceutical Research 2004). Freeze-dryers are commercially available for example from HOF, FTS Systems, Skadi or GEA lyophil.

The term "freeze dried" refers to any biomolecule solution that was treated by the method of "freeze-drying" as described above.

The term "vacuum" refers to a pressure less than atmosphere pressure, such as 500 mbar or less, 300 mbar or less, 200 mbar or less, 100 mbar or less, typically between 0.1 and 1 mbar. The person skilled in the art is aware of different methods of measuring pressure and is thus also familiar with conversion of pressure units, e.g. conversion of the mbar values given herein to corresponding mTorr values, or to atm values, etc.

In a specific embodiment, step (a) comprises providing the dry or solid biomolecule in a container under a reduced gas pressure of 100 mbar or less, 200 mbar or less, 300 mbar or less, or 500 mbar or less.

In another embodiment, step (b) comprises exposing the dry or solid biomolecule in the container to the gas so that the biomolecule is present in an atmosphere of the gas.

Typically step c) comprises sealing the container so that the biomolecule is kept in the gas atmosphere. The container may be hermetically sealed in order to store the biomolecule under sterile conditions.

In one embodiment, the biomolecule is a protein.

In a specific embodiment, the biomolecule is an antibody.

The term "protein" used herein interchangeably with the terms "polypeptide", "peptide" and "oligopeptide", includes all possible forms of that protein, such as mutated forms (comprising one or more mutations), truncated forms, elongated forms, fusion proteins comprising the protein or fragments of the protein, tagged proteins or variants, in which a particular domain is removed or partially removed. The term "protein" may further include monomers, oligomers and polymers of that polypeptide.

The term "antibody" includes polyclonal antibodies, monoclonal antibodies (mAbs), recombinant antibodies, chimeric antibodies, CDR-grafted antibodies, humanized antibodies, fully human antibodies, single chain antibodies, bispecific antibodies, antibody-fusion proteins (such as Ig chimeras), Lama antibodies and/or nano-bodies, as well as fragments (e.g. Fab, Fab', F(ab')₂ and F(v) antibody fragments) including variants and derivatives thereof, provided by known techniques, including but not limited to enzymatic cleavage, peptide synthesis or recombinant techniques.

In one embodiment, the antibody is an antibody directed against the CD20 antigen. An example for a fully humanized monoclonal anti-CD20 antibody is Veltuzumab. Another exemplary anti-CD20 antibody is Rituximab.

In another embodiment, the method of treating a dry biomolecule further comprises a step (d) of reconstitution in order to obtain a physiological acceptable solution. Step (d) may include adding a reconstitution solvent selected from the group consisting of water, buffer, physiological NaCl, Ringer solution, 5% glucose and/or PBS.

The term "reconstitution of a dry biomolecule" may refer to a process of contacting the dry biomolecule with a solvent. This process is usually carried out in a hermetically sealed package or container. For example, the lid of a vial containing the dry biomolecule may be pierced by a needle of a syringe filled with a solvent and the solvent may then be introduced into the vial.

The reconstituted biomolecule solution may be a formulation further containing one or more components selected from the group consisting of a stabilizer, a bulking agent, an osmolyte, a buffering agent, a detergent and the like.

Typically, the methods of the present invention are suitable for a quick reconstitution of the biomolecule, e.g. by avoiding excessive formation of stable foam. Further, in the methods of the present invention the dry biomolecule, also referred to as cake, may be wetted quickly, collapses quickly or even immediately and/or dissolves quickly. Further, there are typically no or only little gel-like particles and/or solid particles of the biomolecule visible after reconstitution of the dry biomolecule. In one embodiment, the foam layer decays or collapses quickly or even immediately. For example, the foam layer decreases to levels that no or only few bubbles are left within 35 minutes, within 20 minutes or within 15 minutes after adding of the reconstitution solution. In addition or alternatively, the biomolecule may be dissolved within 10 minutes so that no or only very few gel-like particles and/or solid particles are visible.

The term "physiological acceptable" typically means that the solution when administered to humans, usually does not induce allergic, toxic or similar unwanted reactions. Such a physiologically acceptable solution may have a pH ranging from 4 to 10, 5 to 9, or from 6 to 8.

Usually the reconstituted biomolecule solution is isotonic. The solution has the same osmotic pressure as the blood of a mammal to which the solution is administered.

Typically, the reconstituted biomolecule solution is suitable for parenteral or oral administration to human patients. Thus, the reconstituted biomolecule solution may be a pharmaceutical dosage form or pharmaceutical formulation. E.g., the solution may be suitable for subcutaneous or oral administration.

Usually, the reconstituted biomolecule solution has a high concentration of the biomolecule. For example, the concentration may be in the range from between 30 mg/ml and 300 mg/ml or from between 60 mg/ml and 200 mg/ml or from between 80 mg/ml and 150 mg/ml.

Another aspect of the invention is a method for reconstituting a dry biomolecule, comprising the following steps:
a) providing a dry biomolecule in a gas atmosphere of a gas selected from the group consisting of carbon dioxide, nitrous oxide, hydrofluoroalkane and/or mixtures thereof,
b) adding a reconstitution solution to the dry biomolecule.

The dry biomolecule may be provided in a sealed container. The solution may be added using standard aseptic techniques, such as the introduction of a needle of a syringe. The reconstitution may be completed if the biomolecule is essentially dissolved so that there are essentially no visible solid or gel-like particles left.

Further, there is typically no or only little foam visible. In particular, excessive foam formation and/or the formation of stable foams may be avoided by the methods described herein.

The term "foam formation" may refer to the building of bubbles upon reconstitution, when the solvent contacts the biomolecule.

The term "stable foam" may refer to foam that collapses slowly. For example, with stable foam the time period from solvent addition until essentially no foam is visible may last at least 20 minutes, at least 30 minutes, at least 40 minutes or at least 60 minutes.

The term "excessive foam formation" may refer to the formation of a foam layer upon adding the diluents to the dry biomolecule, wherein the volume of the foam layer may be at least 1/5, at least 1/3 or at least 1/2 of the volume of the diluents.

In a further embodiment, step (a) of the method for reconstituting a dry biomolecule comprises conducting the method for treating a dry biomolecule as described in any of the embodiments above.

The methods of the present invention may be carried out under sterile conditions.

A further aspect of the invention is the use of a gas selected from the group consisting of carbon dioxide, nitrous oxide, hydrofluoroalkane and/or mixtures thereof for reducing foam formation upon reconstitution of a dry biomolecule, wherein a reconstitution solution is added to the dry biomolecule under an atmosphere of the gas.

Another aspect deals with a container comprising a dry biomolecule in a gas atmosphere of a gas selected from the group consisting of carbon dioxide, nitrous oxide, hydrofluoroalkane and/or mixtures thereof.

The sealed container may contain a dissolution aid, such as an impeller, magnetic stirrer, solids like beads out of inert material such as glass, polyethylene or ceramic.

### Embodiment examples

### Example 1: Reconstitution of dried Veltuzumab

The IgG1 antibody Veltuzumab was provided by Immunomedics, New Jersey, US. L-histidine and sucrose were purchased from Merck, Darmstadt, Germany. 10R lyophilisation vials were purchased from Schott, Mainz, Germany. 20 mm two-legged lyo stoppers were purchased from West, Eschweiler, Germany.

Veltuzumab was formulated in a sucrose - histidine buffer at pH 5.5. The total solid content was about 10%.

| | |
|---|---|
| Veltuzumab | 50.0 mg/mL |
| Sucrose | 120 mM |
| L-Histidine HCl*H20 | 10 mM |
| Polysorbate 20 | 0.10 mg/mL |
| NaOH (2 M) | q.s. |
| Water | ad 1.0 mL |
| pH | 5.5 |

For each of the following exemplary venting gases a volume of 7 mL was filled in 10R vials suitable for lyophilisation. The vials were partly stoppered with a two-leg lyo stopper and placed into the freeze-dryer. The vials can be freeze-dried using a standard programme, e.g. as described in Ed Tappler: Validation of lyophilization: Equipment and Process; in: Biotechnology: Pharmaceutical Aspects Volume II Lyophilization of Biopharmaceuticals, AAPS Press, 2004; which is suitable for all samples and adaptable if desired (Table 1: Freeze-drying standard programme).

**Table 1: Freeze-drying standard programme**

| **No.** | **Description** | **Shelf temperature [°C]** | **Time [min]** | **Pressure [mTorr]** |
|---|---|---|---|---|
| 1 | Loading | +5 | 2 h | atm |
| 2 | Ramp to Freezing | +5 to -40 | 1.5 h | atm |
| 3 | Freezing | -40 | 3 h | atm |
| 4 | Evacuating | -40 | 0.5 h | 80 |
| 5 | Ramp to Primary Drying | -40 to -10 | 1 h | 80 |
| 6 | Primary Drying | -10 | 72 h | 80 |
| 7 | Ramp to Secondary Drying | -10 to +20 | 1 h | 80 |
| 8 | Secondary Drying | +20 | 5 h | 80 |
| 9 | Venting and Stoppering | +20 | n/a | atm/¹⁾ |

| | | | | |
|---|---|---|---|---|
| ¹⁾ pressure of 0.1 mbar; "vacuum" | | | | |

During the lyophilisation process nitrogen was used to control the pressure at 80 mTorr. After step 8, the samples were removed from the freeze-dryer.

In step 9 "venting and stoppering", a part of the samples were loaded again into the freeze-dryer. When the pressure in the dryer reached 80 mTorr the chamber was vented with either argon, helium, carbon dioxide, nitrous oxide or tetrafluoroethane. One reference used nitrogen as vent gas and in another reference the vials were stoppered without venting. After stoppering, the samples were either stored at 2-8°C for 9 months or reconstituted immediately.

For reconstitution, 2.2 ml sterile water for injection was drawn into a 3 ml syringe equipped with a 1 inch 20-gauge needle. The vial containing the dried Veltuzumab antibody was placed upright on a flat surface and by usage of standard aseptic techniques the needle was inserted and the sterile water was injected directly onto the biomolecule. While keeping the vial upright it was gently swirled for approximately 1 minute to evenly wet the powder without shaking. Afterwards, the vial was gently swirled for 5 to 10 seconds approximately every 5 minutes. The concentration of the lyophilisate was 133 mg/ml after reconstitution. The filling weight of the vials was 7.2 g performed with n = 5 vials.

**Table 2: Reconstitution behavior of IgG1/Veltuzumab after storage for 9 months at 2-8°C.**

| **Venting gas** | **foam develop.** | **foam pore quality** | **Foam collapse** | **Cake wetting** | **Cake collapse** | **Cake solution** | **Cake remnants** |
|---|---|---|---|---|---|---|---|
| **Ar** | strong | fine | >40 min | good | < 1 min | <15 min | some |
| **He** | strong | fine | >35 min | good | < 2 min | <20 min | no |
| **CO₂** | medium | coarse | < 10 min | good | immediately | <10 min | no |
| **N₂** | strong | fine | >40 min | good | < 1 min | <15 min | some |
| **vacuum** | minimal | - | immediately | bad | not completely | not completely | many |
| **N₂O** | medium | coarse | <10 min | good | immediately | <10 min | no |
| **HFA 134a** | medium | coarse | <10 min | good | immediately | <10 min | no |

Upon reconstitution with water for injection the dissolving samples showed foam formation due to the gas in the pores of the lyophilisates being displaced by the water. Depending on the type of gas, the extent of foaming and the stability of the foam were different.

In the samples stored for 9 months, nitrogen formed a thick layer of foam with very small bubbles that lasted the longest. Helium and argon formed less thick layers of foam but the foam dissipated almost as slow as the nitrogen foam. Carbon dioxide, nitrous oxide and tetrafluoroethane showed much less foam with a much coarser structure that dissipated rapidly. As expected, the vacuum samples did not show any foam formation. However, the wettability of the lyophilisates from the vacuum was impaired (Table 2: Reconstitution behavior of IgG1/Veltuzumab).

Samples that were reconstituted shortly after venting and stoppering showed the same foam development, foam quality and foam collapse behaviours as the samples stored for 9 months. For all gases the lyophilisation cake wetting, lyophilisation cake collapse, lyophilisation cake solution and lyophilisation cake remnants behaviours were similar in samples immediately reconstituted after stoppering as in samples stored for 9 months. Samples shortly reconstituted after stoppering under vacuum showed good lyophilisation cake wetting, immediate lyophilisation cake collapse and complete lyophilisation cake solution within 10 min. If the liquid has to be extracted against the pull of the vacuum inside the vial, a ventilation step may be required after reconstitution.

Long term stability is not affected regardless of which gas is used. Neither aggregation levels nor chemical modifications are influenced.

### Example 2: BSA

Bovine Serum Albumin (BSA) was purchased from icp biologicals, Auckland, New Zealand. L-histidine and sucrose were purchased from Merck, Darmstadt, Germany. 10R lyophilisation vials were purchased from Schott, Mainz, Germany. 20 mm two-legged lyo stoppers were purchased from West, Eschweiler, Germany.

BSA was formulated in a sucrose - histidine buffer at pH 5.5. The total solid content was about 10%.

| | |
|---|---|
| Bovine serum albumin: | 50.0 mg/mL |
| Sucrose | 120 mM |
| L-HistidineHCl*H2O | 10 mM |
| Polysorbate 20 | 0.10 mg/mL |
| NaOH (2 M) | q.s. |
| Water | ad 1.0 mL |
| pH | 5.5 |

Lyophilisation, storage and reconstitution were carried out as described in Example 1.

**Table 3: Reconstitution behavior of BSA (Lyo.: Lyophilisate) after storage for 9 months at 2-8°C.**

| **Venting gas** | **foam develop.** | **foam pore quality** | **Foam collapse** | **Cake wetting** | **Cake collapse** | **Cake solution** | **Cake remnants** |
|---|---|---|---|---|---|---|---|
| **Ar** | strong | fine | >60 min | good | < 1 min | <20 min | some |
| **He** | strong | fine | >40 min | bad | < 2 min | <25 min | no |
| **CO₂** | medium | coarse | <15 min | good | immediately | <10 min | no |
| **N₂** | strong | fine | >60 min | good | < 1 min | <20 min | some |
| **vacuum** | minimal | - | immediately | bad | immediately | <10 min | some |
| **N20** | medium | coarse | >15 min | good | immediately | <10 min | no |
| **HFA 134a** | medium | coarse | >15 min | good | immediately | <10 min | no |

In the samples stored for 9 months, nitrogen formed a thick layer of foam with very small bubbles that lasted the longest. Helium and argon formed less thick layers of foam but the foam dissipated almost as slow as the nitrogen foam. Carbon dioxide, nitrous oxide and tetrafluoroethane showed much less foam with a much coarser structure that dissipated rapidly. As expected, the vacuum samples did not show any foam formation. However, the wettability of the lyophilisates from the vacuum was impaired (Table 3: Reconstitution behavior of BSA).

Samples that where reconstituted shortly after venting and stoppering and showed the same foam development, foam quality and foam collapse behaviour as samples stored for 9 months. For all gases the lyophilisation cake wetting, lyophilisation cake collapse, lyophilisation cake solution and lyophilisation cake remnants were similar in samples reconstituted immediately after venting and stoppering as in samples stored for 9 months. Samples shortly reconstituted after stoppering under vacuum showed good lyophilisation cake wetting, immediate lyophilisation cake collapse, and complete lyophilisation cake solution within 10 min. If the liquid has to be extracted against the pull of the vacuum inside the vial, a ventilation step may be required after reconstitution.

Carbon dioxide, nitrous oxide and tetrafluoroethane showed improved wettability of the lyophilisate and the coarse texture of the less intense foam allows the health care personnel to decide much quicker whether reconstitution is complete or not. Excessive foaming of highly concentrated protein lyophilisates can be avoided and reconstitution can be significantly improved by venting the lyophilisation chamber with carbon dioxide, nitrous oxide, tetrafluoroethane or a mixture thereof instead of nitrogen before stoppering.

## Claims

1. Method for treating a dry biomolecule, comprising the following steps:
a) providing a dry biomolecule in a container,
b) exposing the dry biomolecule in the container to a gas selected from the group consisting of carbon dioxide, nitrous oxide, hydrofluoroalkane and/or mixtures thereof and
c) optionally, closing the container.

2. Method according to claim 1,
wherein the dry biomolecule is a freeze dried biomolecule.

3. Method of claim 2,
wherein step a) comprises freeze-drying the biomolecule.

4. Method according to any of the preceding claims,
wherein step a) comprises providing the dry biomolecule in a container under a reduced gas pressure of 500 mbar or less, 200 mbar or less, or 100 mbar or less.

5. Method according to any of the preceding claims,
wherein step b) comprises exposing the dry biomolecule in the container to the gas so that the dry biomolecule is present in an atmosphere of the gas.

6. Method according to any of the preceding claims,
wherein step c) comprises sealing the container so that the dry biomolecule is kept in the gas atmosphere.

7. Method according to any of the preceding claims,
wherein the biomolecule is a protein.

8. Method according to any of the preceding claims,
wherein the biomolecule is an antibody.

9. Method according to any of the preceding claims,
wherein the container is selected from the group consisting of a vial, an ampoule, a carpule, a cartridge, a syringe and a double chamber system.

10. Method according to any of the preceding claims,
further comprising a step d) of reconstitution, optionally with a solvent selected from the group consisting of water, buffer, physiological NaCl, Ringer solution, 5 % glucose and/or PBS, in order to obtain a physiological acceptable solution.

11. Method according to claim 10, wherein the reconstituted biomolecule solution is isotonic.

12. Method according to claims 10 and 11,
wherein the reconstituted biomolecule solution is suitable for parenteral or oral administration to human patients.

13. Method according to claims 10 to 12,
wherein the reconstituted biomolecule solution has a concentration of from between 30 mg/ml and 300 mg/ml, from between 60 mg/ml and 200 mg/ml or from between 80 mg/ml and 150 mg/ml.

14. Method according to any of the preceding claims, wherein the hydro fluoro alkane is 1,1,1,2-tetrafluorethane.

15. Method for reconstituting a dry biomolecule, comprising the following steps:
a) providing a dry biomolecule in a gas atmosphere of a gas selected from the group consisting of carbon dioxide, nitrous oxide, hydrofluoroalkane and/or mixtures thereof,
b) adding a reconstitution solution to the dry biomolecule.

16. Method according to claim 14,
wherein step a) comprises conducting the method of any of claims 1 to 9.

17. Method according to claims 14 or 15,
wherein the hydro fluoro alkane is 1,1,1,2-tetrafluorethane.

18. Use of a gas selected from the group consisting of carbon dioxide, nitrous oxide, hydrofluoroalkane and/or mixtures thereof for reducing foam formation upon reconstitution of a dry biomolecule by adding a reconstitution solution to the biomolecule under an atmosphere of the gas.

19. Use of claim 18,
wherein the hydro fluoro alkane is 1,1,1,2-tetrafluorethane.

20. Container, comprising a dry biomolecule in an atmosphere of a gas selected from the group consisting of carbon dioxide, nitrous oxide, hydrofluoroalkane and/or mixtures thereof.

21. Container of claim 20,
wherein the hydro fluoro alkane is 1,1,1,2-tetrafluorethane.
